# EUROPÄISCHE PATENTSCHRIFT

(11) **EP 1 308 169 B1**
(45) Veröffentlichungstag und Bekanntmachung des Hinweises auf die Patenterteilung: **08.06.2005**
(21) Anmeldenummer: 02024438.0
(22) Anmeldetag: 29.10.2002
(51) Int. Cl.: A61K 47/08, A61K 47/14, A61K 47/26, A61K 47/28, A61K 47/34, A61K 31/4045

(54) **Topisch applizierbare Zusammensetzungen mit externer Wirkstoffdepotbildung, deren Herstellung sowie deren Verwendung**
Reservoir composition for the topical application of sparingly soluble drugs, their production and use
Composition-réservoir pour admistration topique de composées peu solubles dans l'eau, leur préparation et usage

(30) Priorität: 06.11.2001 DE 10154324
(43) Veröffentlichungstag der Anmeldung: 07.05.2003
(73) Patentinhaber: Merz Pharma GmbH & Co.KGaA, 60318 Frankfurt (DE)
(72) Erfinder: Beutler, Rolf D., Dr., 64739 Höchst/Hummetroth (DE); Nürnberg, Eberhard, Prof. Dr., 91080 Uttenreuth/Weiher (DE); Schrader, Karl-Heinz, Dr., 37603 Holzminden (DE); Schmidt, Karl-Heinz, Prof. Dr. Dr., 72810 Gomaringen (DE)
(74) Vertreter: Müller, Claudia, Dr.

(56) Entgegenhaltungen:
- WO-A-01/32115
- WO-A-98/17315
- US-A- 5 326 566
- US-A- 5 508 039
- US-A- 5 720 948
- US-A- 6 019 997
- KANDIMALLA K K ET AL: "Optimization of a vehicle mixture for the transdermal delivery of melatonin using artificial neural networks and response surface method" JOURNAL OF CONTROLLED RELEASE, ELSEVIER SCIENCE PUBLISHERS B.V. AMSTERDAM, NL, Bd. 61, Nr. 1-2, 27. August 1999 (1999-08-27), Seiten 71-82, XP004362965 ISSN: 0168-3659
- DATABASE WPI Section Ch, Week 200126 Derwent Publications Ltd., London, GB; Class B02, AN 2001-257591 XP002231272 & WO 01 13950 A (TAKEDA CHEM IND LTD), 1. März 2001 (2001-03-01)

## Beschreibung

Die vorliegende Erfindung betrifft topisch applizierbare Zusammensetzungen, welche gekennzeichnet sind durch einen Gehalt an einem oder mehreren schwerlöslichen Wirkstoffen, einem oder mehreren wasserlöslichen /wasserdispergierbaren nicht ionischen Tensiden mit einem HLB- Wert von mindestens 8, nämlich Derivate des Polyoxyethylens (=POE) und welche Wasser oder ein Wasser-Alkohol-Gemisch aufweisen.
Mit solchen Zusammensetzungen ist es überraschenderweise möglich, eine externe Wirkstoffdepotbildung insbesondere bei der Anwendung herbeizuführen. Dadurch können unerwünschte Nebenwirkungen, welche normalerweise durch die Verabreichung solcher Wirkstoffe in systemischer, also resorbierender Form auftreten, vermieden und eine lokal zielgerichtete Wirkstofffreisetzung ermöglicht werden.

### Stand der Technik

Bisher werden zur Hautbehandlung verschiedene galenische Darreichungsformen, d.h. unterschiedliche physikalisch-chemische Systeme eingesetzt. Normalerweise handelt es sich um streichfähige Zubereitungen, die in Form von Salben, Cremes, Pasten oder auch Pudern verwendet werden. In speziellen Fällen ist auch die Anwendung von flüssigen Darreichungsformen angezeigt, die als Lösungen, Tinkturen, Lotionen, Gele mehr oder weniger große Anteile an Alkoholen, wie Ethanol oder 2-Propanol, enthalten.
Die einzelnen Applikationsformen sind hinsichtlich ihrer Wirksamkeit und Anwendungsfähigkeit unterschiedlich erfordern üblicherweise eine große Anzahl unterschiedlicher Hilfsstoffe zur Stabilisierung.
Dies impliziert vielfach ein unerwünschtes Hautgefühl sowie ggf. auch sensibilisierende/allergische Reaktionen, welche aufgrund der Vielzahl der unterschiedlichen Hilfsstoff-Komponenten nicht oder nur sehr schlecht noch zuordbar sind. Darüber hinaus ist bei den bisher beschriebenen Formulierungen zur externen Anwendung von einer systemischen Resorption der in der Rezeptur enthaltenen pharmazeutischen bzw. kosmetischen Wirkstoffe auszugehen. Dieser resorptive Effekt ist verbunden mit der systemischen Wirkung, die bei pharmakologisch differenten Stoffen unerwünscht und oft schädlich ist und sogar die Anwendung dieser Substanzen verbieten würde, vgl. Hautarzt, 1999,50,S.5-11.
Zahlreiche Veröffentlichungen betreffen Zusammensetzungen zur Anwendung auf Haut und Haar mit unterschiedlichen Indikationszielen, welche im folgenden näher erläutert werden:
Die PCT/EP00/11976 beschreibt ein Mittel zur Behandlung der androgenetischen Alopezie vom weiblichen Typ, sowie zur Haarbehandlung. Als Wirkstoff wird hier Melatonin eingesetzt. Das Mittel kann als Spray, Lösung, Creme mit üblichen Melatoninmengen verwendet werden. Weiterhin können Hilfsstoffe, sowie gegebenenfalls weitere pharmakologisch aktive Wirkstoffe (z.B. Vitamine) sowie Wasser, Alkohol oder lipophile Substanzen oder Penetrationsverstärker enthalten sein. Es wird insgesamt auf die systemische Wirkung des Mittels abgestellt, da vor allem auch Alkohole die Resorption fördern.
Die EP B 214 245 betrifft eine topisch applizierbare pharmazeutische Zusammensetzung, wobei eine Kombination aus Melatonin, 5- Methoxytryptamin, 5-Methoxytryptophan, 5- Methoxytryptophol, 5- Methoxyindol-3-essigsäure und 6 Hydroxymelatonin vorliegt. Die Zusammensetzung in Form von Cremes, Lotionen etc. mit Ausnahme von wässrigen Lösungen ist zur Konditionierung von menschlicher Haut und zur Verbesserung ihrer kosmetischen und physischen Erscheinung gedacht (z.B. Seborrhoe/Akne-Bekämpfung),wobei auf den resorptiven Effekt Bezug genommen ist. Dieser ist wie erwähnt verbunden mit der o.g. systemischen Wirkung. Es wird ferner eine Anti-Akne-Lotion beschrieben, welche neben Wasser Emulgatoren sowie lipophile Hilfstoffe wie Isopropylstearat, Isocetylstearat, Cetylalkohol und Melatonin aufweist, wobei insbesondere auf die absorptive (resorptive) Wirkung hingewiesen wird.
In der EP B 343 223 ist die systemische Behandlung oder Prävention von metabolischen Alterserscheinungen. durch Verwendung von Melatonin und Derivaten hiervon zur Herstellung einer pharmazeutischen Zusammensetzung beschrieben. Eine spezielle Art der Arzneimittelformulierung oder Mengenangaben des Wirkstoffes sind hier nicht angegeben. Es wird jedoch auf den erhöhten Spiegel von T3 und T4 im Blut nach chronischer Behandlung von älteren Mäusen mit Melatonin in Trinkwasser hingewiesen, was die systemische, also Resorptionswirkung aufzeigt, vgl. auch die o.g. Literatur "Hautarzt, 1999".
Die EP B 215 423 offenbart eine transdermal resorbierbare wasserhaltige Zubereitung von Arylpropionsäuren, welche 1- 15 Gew.-% dieser Wirkstoffe, 10- 40 Gew % Polyoxyethylen- Polyoxypropylen- Blockpolymere (z.B. Pluronic ® F 127) sowie 10- 50 Gew%. eines oder mehrerer physiologisch verträglicher organischer Lösungsmittel und mindestens 10 % Wasser sowie bis zu 1 Gew. %. Konservierungsstoffe, Farbstoffe und/oder Parfüm aufweist. Als physiologisch verträgliche Lösungsmittel werden Ethanol, 2-Propanol, 1,2-Propandiol, Glycerin unter anderem genannt. Mit solchen Zusammensetzungen soll eine wesentlich verbesserte und beschleunigte Resorption der Wirkstoffe erreicht werden.
JP 61212512 A2 betrifft ein Haarwuchsmittel aus 0,05% Melatonin, 10 / EtOH, 37,95% H₂O, 2% hydriertes Rizinusöl; EP A 820767 offenbart Zusammensetzungen mit Lipiden wie z.B. Octyldecanol, Sonnenblumenöl, Sepigel 305, Cyclomethicon sowie Glycerol, Lubragel, PEG 100/20 Stearate und weiteren Stoffen sowie Melatonin zur Hautdepigmentierung; (US Patent 5932 608).
EP 851 855(WO 97/06140) betrifft pharmazeutische Zusammensetzungen, enthaltend Melatoninderivate , welche als Antidepessiva in üblichen, insbesondere oral und parenteral verabreichbaren Formen, wirken sollen. Da Bezug genommen wird auf bisher bekannte Zubereitungsformen, muß auch hier von der systemischen Wirkung ausgegangen werden, Die US Patent 6093 409 beschreibt kosmetische/dermatologische Zusammensetzungen als Anti- Aging- Mittel, enthaltend neue Melatoninderivate und insbesondere Lipophile zur Herstellung von Emulsionen oder Lotionen, wodurch die bisher beschriebene systemische Wirkung hervorgerufen wird.
EP-B 796 080 betrifft ein Kombinationspräparat aus Provitamin A, B-Vitaminen, Vitamin C + E, Coenzym Q 10, Methionin/Cystein, welches, wenn es nicht in Tabletten/Kapsel/Dragee-Form vorliegt, zur topischen Verabreichung auch einen Emulgator (z.B. Lecithin) und Öle/Fette sowie weitere Wirkstoffe wie z.B. Melatonin aufweisen kann. Das Produkt ist zur Förderung des Haarwachstums geeignet und entfaltet seine Wirkung systemisch aufgrund der genannten Formulierungsarten.
Die WO 00/48559 betrifft Zusammensetzungen zur Behandlung von Alopezia, enthaltend Nikotinsäure, D- alpha- Tocopherol, ggf. Minoxidil, sowie DMSO und vorzugsweise Alkohol. DMSO ist ein Resorptionsverstärker.
FR 27 53095 A1 beschreibt die Verwendung von Melatonin und dessen Derivaten als entzündungshemmendes Mittel; z.B. als Gel oder Shampoo. Dabei können wässrig/alkoholische Lösungen einerseits sowie Lotionen, Cremes etc. andererseits topisch angewendet werden. Hierzu sind insbesondere eine O/W-Emulsion mit Tween 60, Glycerolstearat und verschiedenen Ölen, ein Shampoo mit einem anionischen Waschmittel (Natriumlaurylethersulfat) und Hydroxypropylcellulose sowie ein Schmerz-Gel mit Wasser, Ethanol, und Hydroxypopylcellulose beschrieben.
In der US- A- 6 019 997 werden Zusammensetzungen beschrieben zur Verstärkung der transdermalen Freisetzung von Wirkstoffen unter Einsatz hierfür geeigneter Emulgatoren wie Polyglykoside, langkettige Alkohole, Polyglycerolester, Carboxylatester, Phospholipide.
Die US. A- 5 720 948 betrifft nicht ionische Tenside und Öle enthaltende Emulsionen zur Verstärkung der Penetration von Wirkstoffen unter Einsatz geeigneter Emulgatoren wie Glycerol Dilaurat und Polyoxyethylen-10 - stearylether.
In der WO 01 32115 werden Zusammensetzungen zur Verabreichung von Huperizin beschrieben, mit welchen dessen Blutplasmaspiegel erhöht werden kann. Für diese beabsichtigte Resorptionserhöhung werden unterschiedlichste Substanzen wie Fettsäuren, deren Salze, Tenside etc. eingesetzt.
Die WO 98 17315 betrifft transdermale / Membran penetrationsfördemde Zusammensetzungen, worin als Permeationsförderer Polyethylenglykolalkylether enthalten ist.
Der oben genannte Stand der Technik offenbart somit Zusammensetzungen, mit welchen insbesondere durch Resorption - wie auch üblich bei pharmazeutischen Zusammensetzungen - eine systemische Wirkung, vor allem auch durch Resorptionsverstärker wie Alkohole, erzielt werden soll. Dabei weisen Lotionen, welche übliche Komponenten wie Fette/Öle und insbesondere ionische Emulgatoren enthalten, oder Cremes oder auch wässrige Zubereitungen insgesamt eine große Anzahl an potentiell allergenen Hilfsstoffen zur Stabilisierung auf.
Aufgrund der systemischen Wirkung ist somit insgesamt eine zielgerichtete, d.h. nur am gewünschten Ort auftretende Aktivität des Wirkstoffs nicht möglich.

### Gegenstand der Erfindung

Aufgabe vorliegender Erfindung ist es daher, topisch applizierbare Zusammensetzungen bereitzustellen, bei welchen keine, d.h. im wesentlichen keine physiologisch relevante Resorption der Wirkstoffe erfolgt und damit die durch eine systemische Wirkung hervorgerufenen unerwünschten Nebenwirkungen der aktiven, in den Stoffwechsel direkt eingreifenden Komponenten vermieden werden können. Ferner soll die Anzahl an Hilfsstoffen möglichst gering gehalten und eine zielgerichtete Wirkstofffreisetzung ermöglicht werden.
Diese Aufgabe wird erfindungsgemäß gelöst durch eine topisch applizierbare Zusammensetzung mit externer Wirkstoffdepotbildung, welche dadurch gekennzeichnet ist, dass sie 0,01-15% eines oder mehrerer schwer lösliche, insbesondere Wasser- oder Wasser-Alkohol schwerlösliche Wirkstoffe, 1 bis 25 Gew.%, vorzugsweise 2 bis 15 Gew.%, eines oder mehrerer wasserlöslicher oder wasserdispergierbarer nicht ionischer Tenside mit einem HLB- Wert von mindestens 8, vorzugsweise 8-22, nämlich Derivate des Polyoxyethylens (=POE), sowie (z.B.5-95%) Wasser oder ein Wasser-Alkohol-Gemisch aufweist.

Überraschenderweise wurde gefunden, dass mit solchen Zusammensetzungen keine, d.h. im wesentlichen keine physiologisch relevante Resorption der Wirkstoffe erfolgt und somit die damit verbundenen unerwünschten Nebenwirkungen wie Hautaffektion, Irritation vermieden werden können. Ferner kann die Hautverträglichkeit verbessert werden, da lipophile Hilfsstoffe wie Cetylalkohol, Glycerolstearat oder Ester höherer Fettsäuren bzw. Wachse nicht vorhanden sind. Insgesamt erfolgt, auch insbesondere bei der Anwendung, eine Gelbildung, die zur Ausbildung von kohärenten Tensidassoziaten, z.B. Bilayerschichten, führt, welche eine Resorptionshemmung durch eine externe Depotbildung des Wirkstoffs herbeiführt. Aus dem Depot können der oder die Wirkstoffe lokal gezielt in die Haut diffundieren und ihre Aktivität entfalten (no adverse effect level).
Dies war auch deshalb überraschend, da Tenside und auch Alkohole allgemein als Resorptionsförderer bekannt sind, wie vorstehend beschrieben.
Das oder die Tenside können erfindungsgemäß wie erwähnt in Mengen von insbesondere von 1-25% und ganz bevorzugt 5-20% oder auch bevorzugt 2-15% und die Wirkstoffe in einer Menge von 0,01-15%, insbesondere 0,01-10%, vorzugsweise 0,1-10% und insbesondere 0,1-7% oder auch 0,01-5% vorhanden sein. Bevorzugt weist die Zusammensetzung weiterhin 0-30%,vor allem 0-20%, insbesondere 0,1-20% und ganz besonders 0,1-10%, insbesondere 1-8% Zusatzstoffe auf.
Es ist ferner bevorzugt, wenn der Alkohol im Wasser Alkohol-Gemisch ausgewählt ist aus Ethanol, 2-(lso-)Propanol, 1-(n-)Propanol oder Mischungen hiervon, wobei Ethanol oder Ethanol im Gemisch mit 2-Propanol im Verhältnis von 5:1 bis 1:5 bevorzugt sind.
Somit können die Zusammensetzungen 5-95%, oder 10-90%, insbesondere 25-80% und ganz besonders auch 30-75% Wasser oder Wasser-Alkohol-Gemisch enthalten. Dies hängt von der jeweiligen Menge an Wirkstoff(en), Tensid(en) und gegebenenfalls vorhandenen Zusatzstoff(en) ab.
Das oder die Tenside sind insbesondere ausgewählt aus POE- Polyoxypropylen-Blockpolymeren, insbesondere aus Blockpolymeren des Polyoxyethylens und Polyoxypropylens des Poloxamer-Typs. Ganz besonders geeignet sind hierbei Polyoxyethylen- Polyoxypropylen-Blockpolymer- Tenside (Poloxamere) mit einer relativen Molmasse von 1000-18000 ,insbesondere 2000-15000 mit einem Verhältnis von Polyoxyethylen- zu Polyoxypropylen- Einheiten von 95:5 bis 30:70.
Ganz besonders geeignet sind Poloxamere der oben genannten Art mit einem mittleren Molekulargewicht von 2000-15000.
Der oder die Wirkstoffe sind vorzugsweise ausgewählt aus der Gruppe, umfassend Hormone und hormonwirksame Stoffe, antimikrobiell und antiphlogistisch wirksame Substanzen, Vasoaktive Substanzen, Naturstoffe auf pflanzlicher oder synthetischer Basis, Antioxidativ oder Zellschützende Wirkstoffe, Dermotherapeutika, Analgetika, Antiparasitosa/Antiskabiosa, Wundreinigungsmittel, Entzündungshemmer, Antihistaminika, Zytostatika, Cytokine, Immunsuppressoren und Kombinationen hiervon.
Besonders bevorzugt sind Dermotherapeutika, Entzündungshemmer, Analgetika, Antimikrobielle Mittel, Hormone und hormonwirksame Stoffe, durchblutungsfördernde Stoffe und Kombinationen hiervon.
Insbesondere sind hier als Wirkstoff Minoxidil, Aminexil, 17-alpha-Estradiol, Kortikosteroide, Melatonin , Melatonin-Derivate, Coffein, Vitamin A,E und Derivate hiervon oder Mischungen hiervon geeignet.
Die Zusatzstoffe in den erfindungsgemäßen Zusammensetzungen sind insbesondere ausgewählt aus mehrwertigen Alkoholen sowie deren Estern, Kolloiden, Konservierungsstoffen, Farbstoffen, Parfümstoffen, Pflegestoffen, UV-Filter sowie indifferenten Salzen und Puffersubstanzen .
Zu diesen Zusatzstoffen gehören vor allem mehrwertige Alkohole wie Glycerol sowie dessen Ester; und als Kolloide depolymerisierte Eiweiß- oder Kohlenhydratprodukte, Kollagenhydrolysate, Polyvinylpyrrolidone (PVP) und Mischpolymerisate hiervon wie Polyvinylpyrrolidon- Polyvinylacetat.
Die Konservierungsstoffe, Farbstoffe, Pflegestoffe, Parfümstoffe, indifferente Salze, Puffersubstanzen und UV-Filter werden nachstehend näher erläutert.
Besonders bevorzugte Zusatzstoffe sind die genannten Alkohole und deren Ester, insbesondere Glycerol sowie die Kolloide , Konservierungsmittel, Salze und Puffersubstanzen.
Eine besonders bevorzugte Zusammensetzung enthält den oder die Wirkstoffe in einer Menge 0,1-8% und das oder die Tenside in einer Menge von 0,5-35%, insbesondere 5-15%, sowie 0,1-20% Zusatzstoffe und als Rest, z.B. 20-75%, 25-60%, insbesondere 25-50% Wasser oder Wasser-Alkohol-Gemisch, z.B. Ethanol und/oder 2-Propanol, mit einem Anteil von 10-70, insbesondere 10-50% Alkohol,
Weitere geeignete Polyoxyethylen-Polyoxypropylen-Blockpolymerisat (Poloxamer) Tenside sind Polysiloxan- Poloxamer- Tenside, insbesondere Dimethicone - Poloxamer-, Verbindungen (Polysiloxan-Polyether-Copolymere), insbesondere des Abil®-Typs.
Im folgenden werden die einzelnen Inhaltsstoffe der erfindungsgemäßen Zusammensetzungen näher beschrieben:

### I.Tenside

Das oder die Tenside sind wasserlöslich oder wasserdispergierbar. Sie können insofern einen HLB- Wert von mindestens 8, insbesondere 8-22, aufweisen.
Insbesondere bevorzugt sind Tenside mit einem HLB- Wert 10-22, insbesondere 10-16, welche im wesentlichen wasserlöslich oder wasserdispergierbar und nicht ionisch sind.
Zu den erfindungsgemäß eingesetzten Tensiden gehören Derivate des Polyoxyethylens (POE). Derivate des POE sind allgemein POE-Fettsäureester des Glycerols, POE Fettsäuren, POE Fettaölkohole (Macrogole).
Zu den erfindungsgemäß eingesetzten POE Derivaten gehören insbesondere POE-Polyoxypropylen- Blockpolymere, insbesondere des Poloxamer- Typs.
Insbesondere bevorzugt sind Polymere wie Polyoxypropylen - Polyoxyethylen-Blockpolymere (INCI-Name: Poloxamere), der allgemeinen Formel I 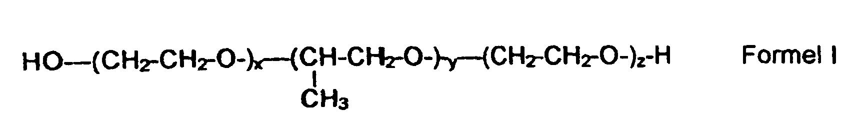 worin x und z jeweils eine Zahl zwischen 2 und 140, insbesondere 10-130, oder 20-100, y Werte zwischen 10 und 60,insbesondere 10-50 bedeuten. Der HLB- Wert beträgt hier insbesondere 8-22, insbesondere 12-22. Insbesondere bevorzugt sind solche Verbindungen der Formel I, worin x=z ist.
Bevorzugt sind solche Produkte mit einer relativen Molmasse von 1000-18000, insbesondere 2000-15000,.und ganz besonders 6000-13000. Dabei beträgt das Verhältnis von Polyoxyethylen(POE-)- zu Polyoxypropylen(POP-)-einheiten bevorzugt 95:5 bis 30:70, insbesondere 85:15 bis 40:60.
Insbesondere gehören zu dieser Gruppe von erfindungsgemäß bevorzugten Tensiden die unter dem Namen Pluronic® bekannten Produkte wie Pluronic® F 127 (HLB:22), Lutrol® F 127 (Poloxamer 407, mit ca. 73% POE ,x=z=98, y=ca.67), Pluronic® PE 6400, (HLB:15), Pluronio® P 65 (HLB:17); Pluronic® P123;(HLB-Wert 8). Weiterhin besonders geeignet sind Pluronic® L44NF (46:56); Pluronic® F 87 NF (72:28); Pluronic® F 68NF (81:19); Pluronic® F 108 NF (82:18) (in Klammem ist das Verhältnis POE zu POP angegeben). Weitere POE- Polyoxypropylen- Blockpolymere (Poloxamer) sind Polysiloxanpoloxamertenside.
Die Poloxamer- Polysiloxan-Tenside, insbesondere Polysiloxan-Polyether-Copolymere, können durch die allgemeine Formel II dargestellt werden: 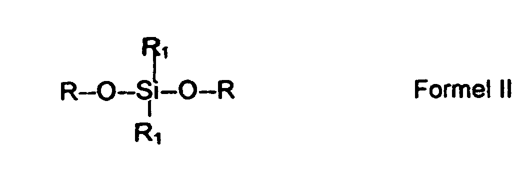 worin R mit Poloxamerresten (wie oben beschrieben) substituierte Silikonbausteine und R₁ bevorzugt Alkyl, insbesondere Methyl, darstellen. Die Poloxamerreste entsprechen den oben unter Punkt 4 genannten Strukturen mit entsprechendem Verhältnis von POE zu POP im Poloxamerteil, der dann die Wasserlöslichkeit bzw. Dispergierbarbeit bedingt.
Insbesondere bevorzugt sind hierunter Dimethicon-Poloxamer-Tenside, insbesondere des Abil®-Typs. Diese können durch die Formel IIb dargestellt werden: 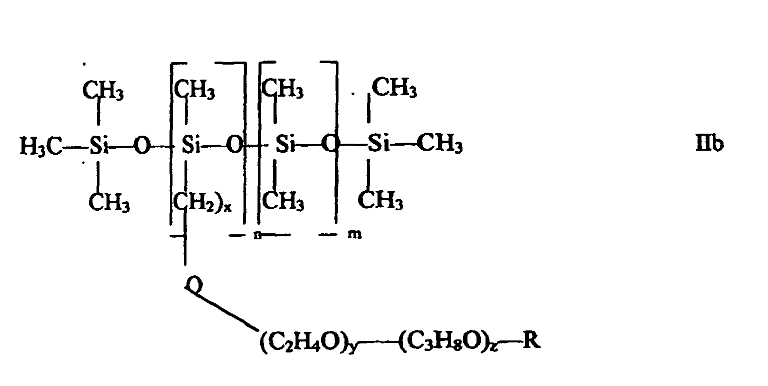 worin R insbesondere Wasserstoff, z und y die o.g. Werte für Formel I, x 1-kleiner 18, vorzugsweise 16, insbesondere 1-10, bedeuten, vgl. H.P. Fiedler, Lexikon der Hilfsstoffe für Pharmazie, Kosmetik und angrenzende Gebiete, 4.Auflage, 1996, S.91. Beispielhaft können solche aus der Reihe der Abil® B 88- Reihe wie Abil® B 8842 (100:0), Abil® 8843; Abil® 8817; Abil® B 8851 (75:25); Abil® 8851 (75:25);Abil® B 8852 (20:80); Abil® 8863 (40:60); Abil® 8873 (35:65); Abil® B 88183 (77:23), Abil®B 183 Abil®B 184 bzw. heute vertriebene entsprechende Produkte anderer Handelsbezeichnungen genannt werden.

Besonders bevorzugte Emulgatoren sind nicht ionische Tenside wie die genannten Poloxamere gemäß Formel I und als Pluronics® bezeichneten Tenside (wie z. B. Lutrol® F 127). Es können auch Emulgatorgemische aus Komponenten der genannten Gruppe eingesetzt werden.

### II:Wirkstoffe

Die erfindungsgemäße Zusammensetzung weist einen Anteil an Wirkstoffen bzw. deren physiologisch verträgliche Abkömmlinge wie Salze, Amide, Ester auf, welche schwerlöslich in Wasser- bzw. Wasser-Alkohol bzw. lipophil sind und normalerweise leicht durch die Zellmembran diffundieren können. Hierzu gehören insbesondere:
Hormone und hormonwirksame Stoffe wie z. B. Sexualhormone vom Typ der Estrogene und Androgene sowie Abkömmlinge und Metabolite hiervon wie 17-alpha-Estradiol; Estradiolbenzoat, Antiandrogene wie Cyproteronacetat, Chlormadinnonacetat, Medroxyprogesteronacetat, Megesterolacetat, Spironolacton, Cimetidin; Melatonin und dessen Derivate, Thymopoietin und Analoga sowie Kombinationen hiervon;
Antimikrobiell wirksame Substanzen wie Antibiotika, Antimykotika(Fungizide) oder antivirale Stoffe, wie beispielsweise Fusidinsäure,Guanidin und Derivate hiervon, lodpovidone, Ketoconazol, Climbazaol, Polidocanol,Gentamycin, Nystatin,Aciclovir;Ciclopirax, Chlortetracyclinhydrochlorid, Ciclopirox, Amorolfin, Noftidin, Itraconazol, Undecylensäure, Mittel gegen Warzen wie Dithranol oder Podophyllin, Podophyllotoxin und Kantharidin, Cyclosporine wie Cyclosporin A, Chlorhexidinhydrochlorid, Dequaliniumchlorid, 2,4-Aminopyrimidin, Thiocyanate und Kombinationen hiervon;
Wundreinigungsmittel wie Kollagenasen, Peptidasen, Trypsin, Streptokinasen, Desoxyribonukleinasen.
Antiphlogistische(auch antibakteriell wirksame) Substanzen wie Glycyrrhizinsäure oder Glycyrrhetinsäure und deren Derivate, z.B. StearylGlycyrrhetinate, Pantothensäurederivate, z.B. Panthenyltriacetat, Allantoin, Bisabolol, Azulene, z.B. Cham-Azulene oder Guaj-Azulen, Triclosan, Chlorhexidin-Derivate; und/oder Piroctone Olamine, Aminexil.
Vasoaktive Substanzen wie Minoxidil; Nikotinsäurederivate wie Methyl- oder Tocopherylnikotinat, alpha- und beta- Hydroxysäuren und deren Derivate, z.B. Salicylsäure, Isopropylbenzylsalicylate,
Naturstoffe auf pflanzlicher oder synthetischer Basis, insbesondere in Wasser schwerlösliche Extrakte z.B. von Salbei, Rosmarin, Orangen, Lavendel, Limetten, Zimt, Geranium, Zedernholz, Roßkastanie, Ginseng, Eisenhut, Ingwer, Brennessel, Birke, Sägezahnpalmfrucht, Dattelkernen, Aesin, RutinoideRosenholz, Baldrian, Ylang-Ylang, Eucaplyptus, Minze, Lemongras, Zypressen, Niaouli, Fichtennadel, Kiefernnadel, Lindenblüten, Campher, Menthol, oder auch Pflanzenextrakten wie Aloe-Vera-Extract, Lindenblüten-Extract, Centella asiatica Extrakt, Efeublätter Extrakt.
Entzündungshemmer wie Diclophenac-Kalium, Indomethacin, Diflunisal, Kortikosteroide wie Clobetason, Kortison, Clobutinol, Betamethasonacetat; Hydrokortison und dessen Ester, Triamcinolonacetonid, Fluprednilydenacetat.
Antihistaminika und antiallergisch wirksame Stoffe wie Diphenhydramin; oder
Antioxidativ sowie zellschützend wirkende Stoffe wie Flavonoide, z.B. Rutin, Ferulasäure und deren Ester oder Coenzym Q 10, oder auch Butylhydroxytoluol, Butylhydroxyanisol, Ascorbylpalmitat, Tocopherol, evtl. in Kombination mit Synergisten wie in Controx® VP (Tocopherol,Lecithin, Ascorbyl Palmitate, Hydrogenated Palm Glycerides Citrate), Gallussäurealkylester wie Octyl-, Dodecyl- und Cetylgallat oder Vitamin A, E, C bzw. deren Derivate (sofern schwerwasserlöslich), z.B. Vitamin A-säure, Retinol, -acetat oder -palmitat, Carotinoide, Tocopherol oder -acetat, Ascorbylpalmitat , Biotin, Mikronährstoffe wie z.B. hydrophobe essentielle Aminosäuren wie Cystin, Cystein, Valin, Leucin, Isoleucin, Phenylalanin, Keratinhydrolysate, Spurenelemente wie Zink , Enzyminhibitoren wie Acetazolamid, oder Kombinationen hiervon;
Analgetika wie Nefenaminsäure, Pentazocin, Piroxicam; Coffein, Opioide wie Morphinderivate, Codein, Hydromasplon, Dihydrocodeindihydrogentartat,
Cytokine wie Interferone, z.B. Interferon-alpha, Interferon-alpha 1a bzw. 1b; Interferon-beta-1a bzw.1b; Interferonalpha-2a bzw. 2b, Interferon-gamma 1b; Cytokininduktoren und Immunmodulatoren wie z.B. Tacrolismus.
Immunsuppressiva wie Calcipotriol, Azathioprin.
Antiparasitosa/Antiskabiosa wie z.B. Lindan, Benzylbenzoat, Mesulfen, Crotamiton, Permethrin.
Zytostatika wie Mittel gegen Hauttumore, Methotrexat.
Dermotherapeutika wie Antiaknemittel, Isotretinoin, Cystin, Zinkoxid, Harnstoff Zinkstearat, Mittel gegen Photodermatosen wie Zinkoxid, Titandioxid, Retinoide, Pigmentierungsregulatoren wie Hydrochinon, Dihydroxyaceton, Harnstoff oder Kombinationen hiervon.
Es können auch Kombinationen von Wirkstoffen innerhalb der einzelnen und aus den verschiedenen Gruppen eingesetzt werden.
Somit kann, je nach kosmetischem oder therapeutischem Ziel eine akute, chronische oder altersbedingte Dysfunktion behandelt werden. Hierbei handelt es sich insbesondere um entzündliche, degenerative, proliferative, genetische, traumatische Zustände von Haut, Schleimhaut, Nägel und Haar. Hierzu gehören beispielsweise Alopezie, Atrophie, Hirsutismus, Alterungsprozesse hiervon, insbesondere der Haut, Haarwachstums- und -Beschaffenheitsstörungen, Verletzungen, oder auch Verbrennungen, Verätzungen. Die erfindungsgemäße Zusammensetzung kann manuel, z.B. durch Auftragen oder Versprühen, je nach Konsistenz, angewendet werden.
Insbesondere sind für die Behandlung von Haut, Schleimhaut, Haar und/oder Nägel Antimikrobielle Mittel, zellschützende,antioxidative Stoffe, Dermotherapeutika, Entzündungshemmer, Analgetika, Hormone/hormonwirksame Stoffe, Antiparasitosa, durchblutungsfördernde Stoffe wie beispielsweise Isotretinoin, Diclophenac-Kalium, Nefenaminsäure,17-alpha-Estradiol, Melatonin und dessen Derivate, Minoxidil, Aminexil, Kortikosteroide, Opiode oder Mischungen hiervon geeignet.
Ferner können je nach therapeutischem oder kosmetischem Ziel Kombinationen von Wirkstoffen wie Interferonen, antimikrobiell wirksamen Stoffen, Zytostatika, zellschützenden Wirkstoffen, oder Analgetika vorteilhafter Weise zur Herstellung der erfindungsgemäßen Zusammensetzungen werden.
Insbesondere können als Wirkstoff Melatonin oder Melatonin-Derivate , oder auch Kombinationen hiervon, insbesondere auch mit Minoxidil, 17-alpha-Estradiol eingesetzt werden.
Zur Wirkstoffgruppe der Melatonine gehören solche der allgemeinen Formel III 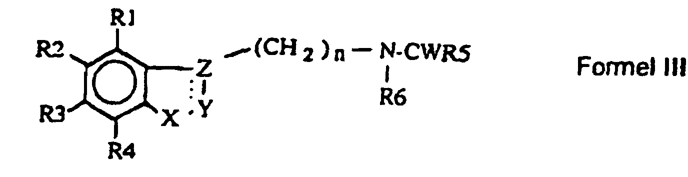 worin n eine Zahl von 1-4, insbesondere 2 bedeutet,
R1, R2, R3 und R4 gleich oder verschieden sind und Wasserstoff, Hydroxyl, C1-C8-Alkyl, C1-C8-Alkoxy, Aryl-C1-C8-Alkyl, Halogen, ausgewählt aus Chlor, Brom. Fluor, Jod, oder Nitro bedeuten,
X Stickstoff, und Z-Y eine c=c- Doppelbindung bedeuten
R5 Wasserstoff, C1-C8-Alkyl, Aryl, oder Aryl-C1-C8-Alkyl und
R6 Wasserstoff, C1-C8-Alkyl, Aryl, oder Aryl-C1-C8-Alkyl bedeuten
und W Sauerstoff bedeutet oder,wenn R5 Wasserstoff ist, die Gruppe CW nicht vorhanden ist..
Insbesondere sind hier Verbindungen bevorzugt, worin R1 Wasserstoff, R2 Hydroxy oder C1-C8Alkoxy, insbesondere C1-C4Alkoxy, insbesondere Methoxy, R3 Hydroxyl oder Alkoxy und R4 Wasserstoff oder R3 Wasserstoff und R4 Hydroxyl oder Alkoxy bedeuten.
Hierunter sind ganz besonders bevorzugt Verbindungen, worin weiterhin Wasserstoff ist ,wenn CW nicht vorhanden ist oder R5 C1-C8Alkyl, insbesondere C1-Alkyl und R6 Wasserstoff oder C1-C4Alkyl bedeutet.
Hierunter sind ganz besonders Verbindungen bevorzugt, worin n=2 ist. Dazu gehört vor allem Melatonin, N-Acetyl-5-methoxytryptamin, (3-(2-acetylaminoethyl)-5-methoxyindol) entsprechend einer Verbindung der o.g. Formel III, worin R1,R2, R4 R6 Wasserstoff, R3 Methoxy, W Sauerstoff, R5 Methyl und n 2 bedeuten.

Ferner sind Melatonin- Derivate der Formel IV umfaßt: 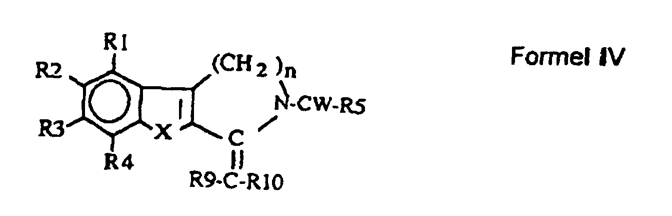 worin R1 bis R5, W , n und X die oben angegebene Bedeutung haben und R9,R10 unabhängig voneinander Wasserstoff, C1-C8Alkyl oder zusammengenommen C3-C8-Cycloalkyl bedeuten.
Bevorzugte Verbindungen sind solche mit n=2, R1,R4=Wasserstoff, R2=C1-C3Alkoxy, insbesondere Methoxy, R3 Hydroxyl, R5 Wasserstoff oder C1-C3-Alkyl, R9/R10 Wasserstoff oder C1-C4-Alkyl.

Die genannten Verbindungen der Formel III und IV (welche im vorstehend genannten Stand der Technik beschrieben sind, z.B. WO97/06140; FR 2753095; US-A 6093409) können insbesondere zur externen Applikation zur nicht systemischen kosmetischen oder therapeutischen Behandlung von der o.g. Zustände, sowie von Haut-Alterserscheinungen, Alopezie, Atrophie, Akne, Seborrhoe, Haarwachstums und Haarbeschaffenheitsstörungen eingesetzt und vor allem auch mit 17-alpha-Estradiol, Spironolacton und/oder Minoxidil kombiniert werden. Insbesondere kann eine Beschleunigung, Förderung oder HemmungNerzögerung des Haarwachstums, Haardickezunahme, Haarschaftproduktionsinitiierung oder - Beschleunigung, Pigmentierung, Bremsung der Androgenwirkung auf den Haarfollikel, Änderung des Wachstums des Haarfollikels herbeigeführt oder Hautverletzungen, Hautpigmentierungsstörungen behandelt werden.

### III:Zusatzstoffe

Daneben können in den erfindungsgemäßen Zusammensetzungen auch Zusatzstoffe in Mengen von 0-30% oder 0-20%, vorzugsweise 1-18% und insbesondere 2-15, bevorzugt 3-10% enthalten sein.
Hierzu gehören:
1) Pflegestoffe wie z.B. Cholesterol, Panthenol, Allantoin, Kamille, Azulene wie Cham- Azulen oder Guja-Azulen oder analoge, dem Fachmann bekannte und gängige Substanzen;
2) Mehrwertige Alkohole und Derivate, insbesondere Ester hiervon wie Glycerol und dessen Ester;
3) Kolloide wie depolymerisierte Eiweiß- oder Kohlenhydrate, Kollagenhydrolysate wie z.B. Dextrine, Dextrane, Povidone, Polyvinylpyrrolidon oder ein Mischpolymerisat hiervon mit Polyvinylacetat;
4) Farbstoffe wie Patentblau, Amidoblau, Orange RGL, Cochenillerot, Chinolingelb, natürliche und synthetische Pigmente;
5) Geruchskorrigentien wie die oben unter "Wirkstoffen" genannten ätherischen Extrakte oder Riechstoffkompositionen;
6) Konservierungsmittel wie Phenoxyethanol, und weiter gebräuchliche Konservierungsstoffe, wie z.B. Formaldehyd und -abspalter, Sorbin- und Dehydracetsäure und deren Salze, Isothiazolinone, Methyldibromoglutaronitril, etc. oder Kombinationen hiervon, oder andere Säuren wie Benzoe- oder Salicylsäure, oder Benzylalkohol oder Ester wie p-Hydroxybenzoesäureester, z.B. Methyl-, Ethyl-, Propyl-, Butyl-, iso-Butylparaben, - oder geeignete Kombinationen der genannten Stoffe;
7) UV-Filter: z.B. Zimtsäureester wie Octyl Methoxycinnamate, Isoamyl p-Methoxycinnamate, Thiazinderivate, Titandioxid oder Zinkoxid;
8) Indifferente Salze und Puffersubstanzen wie Natriumchlorid, Phosphat-Phosphat-Citratpuffer. Hierdurch kann der pH-Wert der Zusammensetzung reguliert und insbesondere auf Haar-, Nägel- Haut-verträgliche Werte eingestellt werden.

Die einzelnen Zusatzstoffe liegen in den dafür üblichen, bekannten Mengen von jeweils 0,01-10%, insbesondere 1-5% vor und können in geeigneter Weise kombiniert werden.
Die genannten Komponenten können auch innerhalb der Gruppen und mit verschiedenen Gruppen kombiniert werden, je nach gewünschtem Zweck.
Insbesondere bevorzugt sind mehrwertige Alkohole und Derivate hiervon in Mengen von z.B. 0,1-10%, und Kolloide sowie Konservierungs- und/oder Geruchskorrigentien, Pflegestoffe, Puffer/Salze.
Wie geschildert, ist es mit den beschriebenen Zusammensetzungen möglich, mit Hilfe der erfindungsgemäßen Tensidassoziate eine Resorptionshemmung der Wirkstoffe durch eine externe Depotbildung hiervon herbeizuführen. Die Kombination Wasser oder Wasser-Alkohol/Tensid führt zu flüssigen bis gelförmigen Externa, wobei insbesondere bei der Anwendung flüssigkristalline Phasen entstehen. Hierdurch entsteht unter Vermeidung unerwünschter Resorptionsprozesse im Sinne einer systemischen Wirkung ein Depot mit inkorporierten Wirkstoffen. Aus diesem heraus kann der oder die Wirkstoffe lokal diffundieren. Damit erfolgt eine gezielte Wirkstofffreisetzung unter Vermeidung von Wirkstoffverlusten und Nebenwirkungen.
Dies ist insbesondere deshalb überraschend, da einerseits die erfindungsgemäß eingesetzten wasserlöslichen oder wasserdispergierbaren Tenside wie auch Alkohole bekanntlich die Resorption fördern und andererseits die Wirkstoffe selbst normalerweise ein gutes Membrandiffusionsvermögen aufweisen.
Insbesondere kann auch auf zahlreiche sonst übliche Hilfsstoffe verzichtet werden, was hinsichtlich der Hautverträglichkeit unter Vermeidung von Irritations- und Schädigungsprozessen für die Haut, Schleimhaut, Nägel und Haar von besonderem Vorteil ist.
Dies kann auch den nachfolgenden Beispielen entnommen werden. So ist im Anwendungsbeispiel 9 ein Vergleich hinsichtlich der Wirkstoff-Resorption (Diffusion durch die Haut)) einerseits und der Diffusion (in die Haut) andererseits beschrieben. Untersucht wurde eine alkoholische Zusammensetzung gemäß Stand der Technik und eine erfindungsgemäße Zusammensetzung am Beispiel des Wirkstoffs Melatonin im Schweinehautmodell. Wie hieraus ersichtlich ist, erfolgt mit dem erfindungsgemäßen, tensidhaltigen Produkt im wesentlichen keine Resorption, jedoch eine Depotbildung (Wirkstoffverbleib auf der Haut:95%) und eine langsame Diffusion des Wirkstoffs aus diesem Depot in die obere Hautschicht, wodurch die aktive Komponente gezielt und ohne Verlust am gewünschten Ort, d.h. nicht systemisch, ihre Wirkung entfalten kann. So können auch unerwünschte Nebenwirkungen vermieden werden.
Bei der Vergleichszusammensetzung allerdings erfolgt eine Resorption, d.h. eine hohe Diffusion durch Haut von ca. 36%. Hieraus ist klar ersichtlich, dass gemäß Stand der Technik eine systemische Wirkung erfolgt. Dieser Befund deckt sich auch mit den von Bangha E. et al. in "Daytime serum levels of melatonin after topical application onto human skin", Pharmacol. 10,298-302(1997) beschriebenen Versuchen, wonach nach topischer Verabreichung von Melatonin in Wasser/Alkohol auf menschlicher Kopfhaut ein erhöhter Melatonin- Plasmaspiegel gemessen wurde, welcher nur durch Resorption des Wirkstoffs entstehen kann und zu einer systemischen Wirkung führt. Im Hinblick auf diese Übereinstimmung lässt sich schließen, dass die Ergebnisse aus vorliegendem Schweinehautmodell auf in- vivo-Verhältnisse übertragbar sind.
Die Erfindung wird anhand der nachfolgenden Beispiele näher erläutert. Hierbei ist in den Beispielen 1 bis 8 die Herstellung der erfindungsgemäßen Zusammensetzungen beschrieben.
In Beispiel 9 ist die Wirksamkeit des erfindungsgemäßen Produktes am Beispiel Melatonin wie oben geschildert im Schweinehautmodell belegt.

### A. Herstellungsbeispiele

### Beispiele 1-6

Die nachfolgend in der Tabelle I beschriebenen erfindungsgemäßen Zusammensetzungen wurden hergestellt, indem Wasser auf 20-90°C, vorzugsweise 20 bis 85°C erwärmt und sodann das oder die Tenside, ggf. zusammen mit temperaturstabilen Zusatzstoffen, unter Rühren bis zur klaren Lösung oder zur Dispergierung inkorporiert, sodann die Mischung auf Raumtemperatur abgekühlt wurde und dann der oder die Wirkstoffe, vorzugsweise gelöst in Wasser oder Wasser/Alkohol, zusammen mit den temperaturlabilen Zusatzstoffen, sofern vorhanden unter Rühren hinzugefügt wurden.

Dabei wurden folgende Produkte gemäß Tabelle I erhalten:

**Tabelle I**

| **Komponente** | **Bsp1** | **Bsp2** | **Bsp3** | **Bsp4** | **Bsp5** | **Bsp6** |
|---|---|---|---|---|---|---|
| Melatonin | 0.1 | 0,01 | 0,01 | 0,01 | 0,01 | |
| Chlorhexidindigluconat | | | | | | 2 |
| Wasser | ad 100 | ad 100 | ad 100 | ad 100 | ad 100 | ad 100 |
| Ethanol, 96% | | 40 | 40 | 40 | 35 | 40 |
| 2-Propanol | 40 | | | | | |
| Glycerin | | | | | 5 | |
| Pluronio® F 127 * | 5 | | | | 5 | 5 |
| Polyglykol 400 ** | | 10 | | | | |
| Abil B 8863 *** | | | 10 | | | |
| Tween 60 **** | | | | 10 | | |

Auf die oben beschriebene Weise wurden auch das erfindungsgemäße Beispiel 7 und das Vergleichsbeispiel 8 hergestellt:

### Beispiel 7

| | |
|---|---|
| Melatonin | 0,5% |
| Ethanol, 96%, | 40,0% |
| dem. Wasser | 54,5 |
| Pluronic F 127 | 5,0 |

### Beispiel 8

| | |
|---|---|
| Melatonin | 0,5% |
| Ethanol, 96% | 40,0% |
| dem. Wasser | 59,5% |

### B. Anwendungsbeispiel

### Beispiel 9

Wie vorstehend beschrieben, wurde die Zusammensetzung gemäß den Beispielen 7 und 8 im Schweinehaut-Modell (Dr. Schrader) untersucht und der Verbleib des Wirkstoffs, hier Melatonin, ermittelt. Dazu wurden aus frischer Schweinehaut, die auf die Hornschicht und die Epidermis reduziert worden war, kreisrunde Stücke ausgestanzt und in Diffusionskammem gespannt. Auf die unverletzte Homschicht erfolgte der Produktauftrag. Während des Versuchs wird die Epidermis mit einer Rezeptorflüsssigkeit umspült und diese in einem geeigneten Gefäß aufgefangen. Anschließend erfolgt die Konzentrationsbestimmung der zu analysierenden Substanz in der Rezeptorflüssigkeit, der Haut und in der Abspülflüssigkeit. So kann die Diffusion durch die Haut bzw. die Depotbildung und die Diffusion in die Haut ermittelt werden. Als Referenzmethode wurde die von Bracher, Faller, Noser entwickelte Methode angewendet, vgl. M.Bracher, C. Faller, F.K. Noser, "Evaluation of an in vitro percutaneous permeation model with two oxidative hair dyes", Int.J.Cosmet.Sci.**9**, 223-226;1986.

Die Ergebnisse sind in folgender Tabelle II dargestellt:

**Tabelle II**

| **Wirkstoffbilanz Melactonin** | **Prozentuale** | **Massenbilanz,** |
|---|---|---|
| | **applizierteDosis, Erf.** | **Vergl.** |
| Verbleib auf der Haut | 95% | 63% |
| Diffusion in die Haut | 3% | 6% |
| Diffusion durch die Haut | 6 Proben; in 5 keine Diff., in einer 1,18% | 36% |

Wie hieraus ersichtlich ist, erfolgt ohne den erfindungsgemäßen Zusatz eine erhebliche Diffusion des Wirkstoffs durch die Haut, während dies überraschenderweise auch schon mit geringen Mengen an erfindungsgemäßem Tensid verhindert werden kann.

## Patentansprüche

1. Topisch applizierbare Zusammensetzung mit externer Wirkstoffdepotbildung, **dadurch gekennzeichnet, dass** sie 0,01-15% eines oder mehrerer Wasser oder Wasser/Alkohol schwerlösliche Wirkstoffe, 1 - 25 % eines oder mehrerer wasserlöslicher oder wasserdispergierbarer Polyoxyethylen - Polyoxypropylen-Blockpolymer Tenside (Poloxamere) mit einem HLB - Wert von mindestens 8 sowie Wasser oder ein Wasser-Alkohol-Gemisch aufweist.

2. Zusammensetzung gemäss Anspruch 1, **dadurch gekennzeichnet, dass** weiterhin 0-20% Zusatzstoffe enthalten sind.

3. Zusammensetzung nach Anspruch 1 oder 2, **dadurch gekennzeichnet, dass** 2 bis 15 Gew. % eines oder mehrerer Tenside enthalten sind.

4. Zusammensetzung gemäss einem der Ansprüche 1 bis 3, **dadurch gekennzeichnet, dass** der Alkohol ausgewählt ist aus Ethanol, 1-Propanol, 2-Propanol oder Mischungen hiervon.

5. Zusammensetzung gemäss einem der Ansprüche 1-4, **dadurch gekennzeichnet, dass** 5-95% Wasser oder Wasser-Alkohol-Gemisch enthalten sind.

6. Zusammensetzung gemäss einem der Ansprüche 1 bis 5, **dadurch gekennzeichnet, dass** als Tensid ein oder mehrere Polyoxyethylen-Polyoxypropylen-Blockpolymer-Tenside mit einer relativen Molmasse von 1000-18000, ausgewählt sind, wobei das Verhältnis von Polyoxyethylen- zu Polyoxypropylen-Einheiten 95:5 bis 30:70 beträgt.

7. Zusammensetzung gemäß Anspruch 6, **dadurch gekennzeichnet, dass** das oder die Tenside ausgewählt sind aus Polyoxymethylen- Polyoxypropylen-Blockpolymeren mit einer relativen Molmasse von 2000-15000 mit einem Polyoxyethylen- zu Polyoxypropylen- Verhältnis von 95:5 bis 30:70.

8. Zusammensetzung gemäss einem der Ansprüche 1-7, **dadurch gekennzeichnet, dass** der oder die Wirkstoffe ausgewählt sind aus der Gruppe, umfassend Hormone und hormonwirksame Stoffe, antimikrobiell und antiphlogistisch wirksame Substanzen, Vasoaktive Substanzen, Naturstoffe auf pflanzlicher oder synthetischer Basis, Antioxidativ-Zellschützende Wirkstoffe, Entzündungshemmer, Antihistaminika, Antiparasitosa / Antiskabiosa, Analgetika, Dermotherapeutika, Zytostatika; Cytokine, Wundreinigungsmittel, Immunsuppressiva und Kombinationen hiervon.

9. Zusammensetzung gemäss Anspruch 8, **dadurch gekennzeichnet, dass** als Wirkstoff Minoxidil, Aminexil, 17-alpha-Estradiol, Kortikosteroide, Melatonin, Melatonin- Derivate, Coffein, Vitamin A,E und Derivate hiervon, oder Mischungen hiervon enthalten sind.

10. Zusammensetzung gemäss einem der Ansprüche 1-9, **dadurch gekennzeichnet, dass** die Zusatzstoffe ausgewählt sind aus der Gruppe, umfassend mehrwertige Alkohole sowie deren Ester, Kolloide, Konservierungsstoffe, Farbstoffe, Parfümstoffe, kosmetische Pflegestoffe UV-Filter sowie indifferente Salze oder Puffersubstanzen.

11. Zusammensetzung gemäss Anspruch 10, **dadurch gekennzeichnet, dass** die Zusatzstoffe ausgewählt sind aus mehrwertigen Alkoholen und deren Estern depolymerisierten Eiweiß- oder Kohlenhydratprodukten, Kollagenhydrolysaten, Povidonen, Dextrinen, Dextranen, Farbstoffen, Parfümstoffen, indifferenten Salzen, Puffersubstanzen und Konservierungsmitteln.

12. Zusammensetzung gemäss einem der Ansprüche 1-11, **dadurch gekennzeichnet, dass** der oder die Wirkstoffe in einer Menge 0,1-8% und das oder die Tenside in einer Menge von 5 - 15%, 0,1-20% Zusatzstoffe sowie als Rest Wasser oder Wasser-Ethanol-Gemisch mit einem Anteil von 10-70% Alkohol vorhanden sind.

13. Verfahren zur Herstellung von Zusammensetzungen gemäß einem der Ansprüche 1-12, **dadurch gekennzeichnet ,dass** man das oder die Tenside und gegebenenfalls vorhandene temperaturstabile Zusatzstoffe der Wasserphase bei 20-90°C hinzufügt, sodann auf Raumtemperatur abkühlt und anschließend den oder die Wirkstoffe vorzugsweise gelöst. In Wasser oder im Wasser-Alkohol-Gemisch zusetzt und sofern vorhanden temperaturlabile Zusatzstoffe inkorporiert.

14. Verwendung von Zusammensetzungen gemäß einem der Ansprüche 1-12 zur Herstellung eines Mittels zur nicht systemisch - topischen kosmetischen oder therapeutischen Behandlung von Haut, Schleimhaut, Haar und Nägel.

15. Verwendung gemäß Anspruch 14, **dadurch gekennzeichnet, dass** die kosmetische oder therapeutische Behandlung akut, chronisch oder altersbedingt erfolgt.

16. Verwendung gemäß Anspruch 14 oder 15, **dadurch gekennzeichnet, dass** entzündliche, degenerative, proliferative, genetische oder traumatische Zustände behandelt werden.

17. Verwendung gemäß Anspruch 16 zur Behandlung von Alopezie, Atrophie, Hirsutismus, Verletzungen, Verbrennungen, Verätzungen, Haarwachstums- oder Haarbeschaffenheitsstörungen, Alterungsprozessen, Nagetwachstums- oder Nagelbeschaffenheitsstörungen.

18. Verwendung gemäß Anspruch 14, **dadurch gekennzeichnet, dass** die Zusammensetzung manuell durch Versprühen oder Einreiben aufgetragen wird..

19. Verwendung von Zusammensetzungen gemäß Anspruch 14, **dadurch gekennzeichnet, dass** als Wirkstoff ein oder mehrere Wirkstoffe, ausgewählt aus Minoxidil, Aminexil, 17-alpha-Estradiol, Kortikosteroiden, Melatonin, dessen Derivaten, Vitamin A, E, Coffein oder Mischungen hiervon enthalten ist zur Herstellung von topisch applizierbaren Zusammensetzungen mit externem Wirkstoffdepot.

## Claims

1. Topically administrable composition with formation of an external active ingredient depot, **characterised in that** it comprises 0.01-15% of one or more active ingredients sparingly soluble in water or water-alcohol, 1-25% of one or more water-soluble or water-dispersible polyoxyethylene-polyoxypropylene block polymer surfactants (poloxamers) with an HLB value of at least 8 and water or a water-alcohol mixture.

2. A composition according to claim 1, **characterised in that** it furthermore contains 0-20% of additives.

3. A composition according to claim 1 or claim 2, **characterised in that** it contains 2 to 15 wt.% of one or more surfactants.

4. A composition according to any one of claims 1 to 3, **characterised in that** the alcohol is selected from among ethanol, 1-propanol, 2-propanol or mixtures thereof.

5. A composition according to any one of claims 1-4, **characterised in that** it contains 5-95% of water or water-alcohol mixture.

6. A composition according to any one of claims 1 to 5, **characterised in that** one or more polyoxyethylene-polyoxypropylene block polymer surfactants with a relative molar mass of 1000-18000 are selected as the surfactant, wherein the ratio of polyoxyethylene to polyoxypropylene units is 95:5 to 30:70.

7. A composition according to claim 6, **characterised in that** the surfactant(s) is/are selected from among polyoxyethylene-polyoxypropylene block polymers with a relative molar mass of 2000-15000 with a polyoxyethylene to polyoxypropylene ratio of 95:5 to 30:70.

8. A composition according to any one of claims 1-7, **characterised in that** the active ingredient(s) is/are selected from the group comprising hormones and hormonally active substances, antimicrobial and antiinflammatory active substances, vasoactive substances, plant-derived or synthetically obtained natural substances, antioxidative cytoprotective active ingredients, antiinflammatories, antihistamines, antiparasitics/antiscabicides, analgesics, dermotherapeutic preparations, cytostatics; cytokines, wound-cleansing agents, immunosuppressants and combinations thereof.

9. A composition according to claim 8, **characterised in that** it contains as active ingredient minoxidil, aminexil, 17-alpha-oestradiol, corticosteroids, melatonin, melatonin derivatives, caffeine, vitamins A, E and derivatives thereof, or mixtures thereof.

10. A composition according to any one of claims 1-9, **characterised in that** the additives are selected from the group comprising polyhydric alcohols and the esters thereof, colloids, preservatives, colorants, perfumes, cosmetic care agents, UV filters and indifferent salts or buffering agents.

11. A composition according to claim 10, **characterised in that** the additives are selected from among polyhydric alcohols and the esters thereof, depolymerised protein or carbohydrate products, collagen hydrolysates, povidones, dextrins, dextrans, colorants, perfumes, indifferent salts, buffering agents and preservatives.

12. A composition according to any one of claims 1-11, **characterised in that** the active ingredient(s) is/are present in a quantity of 0.1-8% and the surfactant(s) in a quantity of 5-15%, 0.1-20% of additives and the remainder as water or water-ethanol mixture with an alcohol content of 10-70%.

13. A process for the production of compositions according to any one of claims 1-12, **characterised in that** the surfactant(s) and optionally present temperature-stable additives are added to the aqueous phase at 20-90°C, the temperature is then reduced to room temperature, after which the active ingredient(s), preferably dissolved in water or in the water-alcohol mixture, is/are added and, if present, temperature-labile additives are incorporated.

14. Use of compositions according to any one of claims 1-12 for the production of an agent for non-systemic/topical cosmetic or therapeutic treatment of the skin, mucous membranes, hair and nails.

15. Use according to claim 14, **characterised in that** the cosmetic or therapeutic treatment is provided on an acute, chronic or age-determined basis.

16. Use according to claim 14 or claim 15, **characterised**
**in that** inflammatory, degenerative, proliferative, genetic or traumatic conditions are treated.

17. Use according to claim 16 for the treatment of alopecia, atrophy, hirsutism, injuries, burns, chemical burns, disorders of hair growth or hair condition, ageing processes, disorders of nail growth or nail condition.

18. Use according to claim 14, **characterised in that** the composition is applied manually by spraying or rubbing in.

19. Use of compositions according to claim 14, **characterised in that** it contains as active ingredient one or more active ingredients selected from among minoxidil, aminexil, 17-alpha-oestradiol, corticosteroids, melatonin, the derivatives thereof, vitamins A, E, caffeine or mixtures thereof for the production of topically administrable compositions with an external active ingredient depot.

## Revendications

1. Composition applicable par voie topique avec formation externe d'un dépôt de principe actif **caractérisée en ce qu'**elle comporte 0,01-15 % d'un ou plusieurs principes actifs peu solubles dans l'eau ou l'eau/alcool, 1-25 % d'un ou plusieurs tensioactifs polymères séquencés polyoxyéthylène-polyoxypropylène (Poloxamères) solubles dans l'eau ou dispersibles dans l'eau ayant une valeur HLB d'au moins 8 ainsi que de l'eau ou un mélange eau-alcool.

2. Composition selon la revendication 1 **caractérisée en ce que** 0-20 % d'additifs sont contenus en outre.

3. Composition selon la revendication 1 ou 2 **caractérisée en ce que** 2 à 15 % en masse d'un ou plusieurs tensioactifs sont contenus.

4. Composition selon l'une des revendications 1 à 3 **caractérisée en ce que** l'alcool est choisi parmi l'éthanol, le 1-propanol, le 2-propanol ou leurs mélanges.

5. Composition selon l'une des revendications 1-4 **caractérisée en ce que** 5-95 % d'eau ou de mélange eau-alcool sont contenus.

6. Composition selon l'une des revendications 1 à 5 **caractérisée en ce que**, comme tensioactif, on choisit un ou plusieurs tensioactifs polymères séquences polyoxyéthylène-polyoxypropylène ayant une masse molaire relative de 1 000-18 000, où le rapport des unités de polyoxyéthylène aux unités de polyoxypropylène est 95 : 5 à 30 : 70.

7. Composition selon la revendication 6 **caractérisée en ce que** le ou les tensioactifs sont choisis parmi les polymères séquencés polyoxyéthylène-polyoxypropylène ayant une masse molaire relative de 2 000-15 000 avec un rapport polyoxyéthylène à polyoxypropylène de 95 : 5 à 30 : 70.

8. Composition selon l'une des revendications 1 à 7 **caractérisée en ce que** le ou les principes actifs sont choisis dans le groupe comprenant les hormones et les substances à action hormonale, les substances à action antimicrobienne et antiphlogistique, les substances vasoactives, les substances naturelles à base végétale ou synthétique, les principes actifs antioxydants-protecteurs des cellules, les anti-inflammatoires, les antihistaminiques, les antiparasitaires/antiscabieux, les analgésiques, les produits dermothérapeutiques, les cytostatiques ; les cytokines, les agents de nettoyage des plaies, les immunosuppresseurs et leurs combinaisons.

9. Composition selon la revendication 8 **caractérisée en ce que** le minoxidil, l'aminexil, le 17-alpha-estradiol, des corticostéroïdes, la mélatonine, des dérivés de la mélatonine, la caféine, les vitamines A,E et leurs dérivés, ou des mélanges de ceux-ci, sont contenus comme principe actif.

10. Composition selon l'une des revendications 1-9, **caractérisée en ce que** les additifs sont choisis dans le groupe comprenant les alcools polyfonctionnels ainsi que leurs esters, les colloïdes, les conservateurs, les colorants, les parfums, les agents pour soins cosmétiques, les filtres UV ainsi que les sels indifférents ou les substances tampons.

11. Composition selon la revendication 10 **caractérisée en ce que** les additifs sont choisis parmi les alcools polyfonctionnels et leurs esters, les produits protéiques ou glucidiques dépolymérisés, les hydrolysats de collagène, les povidones, les dextrines, les dextranes, les colorants, les parfums, les sels indifférents, les substances tampons et les conservateurs.

12. Composition selon l'une des revendications 1-11 **caractérisée en ce que** le ou les principes actifs sont présents en une quantité de 0,1-8 % et le ou les tensioactifs sont présents en une quantité de 5-15 %, 0,1-20 % d'additifs ainsi que le reste d'eau ou de mélange eau-éthanol avec une proportion de 10-70 % d'alcool sont présents.

13. Procédé de production de compositions selon l'une des revendications 1-12 **caractérisé en ce que** l'on ajoute le ou les tensioactifs et les additifs stables à la température éventuellement présents à la phase aqueuse à 20-90°C, puis on refroidit à la température ambiante et ensuite on ajoute le ou les principes actifs de préférence dissous dans l'eau ou dans le mélange eau-alcool et on incorpore les additifs labiles à la température dans la mesure où ils sont présents.

14. Utilisation de compositions selon l'une des revendications 1-12 pour la production d'un agent pour le traitement cosmétique ou thérapeutique non systémique-topique de la peau, des muqueuses, des cheveux et des ongles.

15. Utilisation selon la revendication 14 **caractérisée en ce que** le traitement cosmétique ou thérapeutique se déroule de manière aiguë, chronique ou déterminée par l'âge.

16. Utilisation selon la revendication 14 ou 15 **caractérisée en ce que** des états inflammatoires, dégénératifs, prolifératifs, génétiques ou traumatiques sont traités.

17. Utilisation selon la revendication 16 pour le traitement de l'alopécie, le l'atrophie, de l'hirsutisme, de blessures, de brûlures, d'irritations, de troubles de la croissance des cheveux ou de la structure des cheveux, de processus de vieillissement, de troubles de la croissance des ongles ou de la structure des ongles.

18. Utilisation selon la revendication 14 **caractérisée en ce que** la composition est appliquée manuellement par pulvérisation ou friction.

19. Utilisation de compositions selon la revendication 14 **caractérisée en ce qu'**un ou plusieurs principes actifs choisis parmi le minoxidil, l'aminexil, le 17-alpha-estradiol, les corticostéroïdes, la mélatonine, ses dérivés, les vitamines A, E, la caféine ou leurs mélanges sont contenus comme principe actif pour la production de compositions applicables par voie topique avec un dépôt externe de principe actif.
